**Europäisches Patentamt**

**European Patent Office**

**Office européen des brevets**

(11) Veröffentlichungsnummer: **0 115 624**
A2

(12) **EUROPÄISCHE PATENTANMELDUNG**

(21) Anmeldenummer: 83113044.8

(22) Anmeldetag: 23.12.83

(51) Int. Cl.³: **A 61 B 17/28,** A 61 B 19/00, G 01 L 5/00

(30) Priorität: 07.01.83 DE 8300262 U

(43) Veröffentlichungstag der Anmeldung: 15.08.84
Patentblatt 84/33

(84) Benannte Vertragsstaaten: **AT BE CH DE FR GB IT LI LU NL SE**

(71) Anmelder: **Breger, Gundolf, Dr., Bolohstrasse 127, D-5800 Hagen 1 (DE)**

(72) Erfinder: **Breger, Gundolf, Dr., Bolohstrasse 127, D-5800 Hagen 1 (DE)**

(74) Vertreter: **Schröter, Martin, Dipl.-Ing., im Tückwinkel 22, D-5860 Iserlohn (DE)**

(54) Vorrichtung zum Prüfen der Zugspannung beim Verschieben von Hauptlappen.

(57) Es wird eine Vorrichtung zum Prüfen der Zugspannung beim chirurgischen Verschieben von Hautlappen vorgeschlagen, mit der die dabei auftretende Hauptspannung gemessen werden kann.

Ein solches pinzetten- oder zangenartiges Gerät besitzt breitflächige Enden (11) an zwei Klemmschenkeln (1), wobei diese Klemmschenkel (1) über Zugspannungsmesser (12) einzeln oder gemeinsam kraftschlüssig mit einem Griffteil (2) verbunden sind.

Mit einem solchen Gerät ist es dem Chirurgen möglich, festzustellen, ob die bei der Verschiebung auftretenden Hauptspannungen im zulässigen Bereich liegen, um nach der Vernähung des verschobenen Hautlappenrandes mit einem ortsfesten Hautrand ein Aufreißen der Wundnaht zu vermeiden.

0115624

05.12.1983

1

Bei den verschiedensten chirurgischen Verschiebetechniken während plastischer Operationen werden freigeschnittene bzw. mobilisierte Hautlappen unter Zugspannung in vorgegebener Richtung zur Überdeckung hautloser Partien verschoben. Mit chirurgischen Pinzetten werden diese Hautlappen erfaßt und so unter Zugspannung verschoben, daß die Hautlappenränder etwa parallel zu den Begrenzungsrändern der hautlosen Partie liegen, um danach die parallelen Ränder miteinander vernähen zu können.

BAD ORIGINAL

2

Die auftretende Hautspannung des zu verschiebenden Lappens muß dabei jedoch so gehalten werden, daß die nachträglich auftretende Spannung auf die angebrachten Nahtstellen nicht zu einem Aufreißen der Wundnaht führt, andererseits sollen die zur Freischneidung des zu verschiebenden Hautlappens notwendigen Entlastungsschnitte möglichst klein gehalten werden. Es gehört daher sehr viel chirurgische Erfahrung und Fingerspitzengefühl zur Durchführung dieser Hautverschiebetechniken in optimaler Weise.

Da die Hautspannung des zu verschiebenden Hautlappens von ausschlaggebender Bedeutung hinsichtlich des Heilungserfolges, der Länge des Entlastungsschnittes, des notwendigen Materiales usw. ist, besteht die Aufgabe der Erfindung darin, eine geeignete Vorrichtung zum Prüfen der Zugspannung beim Verschieben von Hautlappen vorzuschlagen.

Gelöst wird diese Erfindungsaufgabe durch eine entsprechende Vorrichtung, die gekennzeichnet ist durch ein pinzetten- oder zangenartiges Gerät mit breitflächigen Enden an den Klemmschenkeln, wobei die Klemmschenkel über Zugspannungsmesser einzeln oder gemeinsam kraftschlüssig mit einem Griffteil verbunden sind.

3

Mit einem solchen erfindungsgemäßen Gerät wird dem Chirurgen eine Vorrichtung an Hand gegeben, mit der er während der Operation die am zu verschiebenen Hautlappen auftretende Zugspannung objektiv feststellen kann. Anhand erkannter zulässiger Zugspannungen ist es ihm damit möglich, die notwendige Länge von Entlastungsschnitten zu bestimmen und bezüglich des Faktors "Hautspannung" die optimale Voraussetzung für den Heilungserfolg zu schaffen.

Eine bevorzugte Ausführungsart der erfindungsgemäßen Vorrichtung ist gekennzeichnet durch zwei an einem Ende gelenkig miteinander verbundene Rohre, in denen jeweils teleskopartig ein Klemmschenkel in Zugrichtung gegen eine im jeweiligen Rohr befestigte Feder mit gleicher Federkennlinie verschiebbar ist, wobei eine mit der Zugkraft korrespondierende Längenänderungsanzeige am Klemmschenkel vorgesehen ist. Eine solche Vorrichtung wirkt in etwa wie eine Federwaage. Anhand der von außen erkennbaren Längenänderung der Klemmschenkel, die mehr oder weniger weit aus den Rohren heraustreten, läßt sich die aufgebrachte Zugspannung, die mit der Hautspannung korrespondiert, feststellen. Mit einer solchen Vorrichtung wird der jeweilige mobilisierte Hautlappen in seine Endverschiebestellung gebracht. Liegt der angezeigte Zugspannungswert unterhalb einer zulässigen Größe, so kann danach die Naht angebracht werden. Die erfindungsgemäße Vorrichtung dient vor allem der Feststellung der auftretenden Hautspannung, gegebenenfalls kann sie aber

BAD ORIGINAL

gleichzeitig operatives Hilfsgerät in der Art einer chirurgischen Pinzette oder Zange verwendet werden.

In weiterer Ausbildung der erfindungsgemäßen Vorrichtung wird eine Feststellvorrichtung für die das Griffteil bildenden Rohre vorgeschlagen. Mit dieser Feststellvorrichtung läßt sich die den Hautlappen erfassende Klemmstellung fixieren. Um nach Öffnung der erfindungsgemäßen Vorrichtung eine Spreizung der Rohre automatisch zu erreichen, kann eine Spreizfeder zwischen den Rohren angeordnet sein.

Nach einem weiteren Vorschlag der Erfindung ist eine Vorrichtung gekennzeichnet durch zwei zangenartig gelenkig miteinander verbundene, in der Klemmstellung festsetzbare Klemmschenkel, von denen ein Schenkel mit einem Ende teleskopartig geführt in einem Griffrohr in Zugrichtung gegen eine Feder verschiebbar und eine mit der Zugkraft korrespondierende Längenänderungsanzeige am Schenkel vorgesehen ist.

Mit einer solchen Vorrichtung wird der zu verschiebene Hautlappen erfaßt und festgeklemmt. Die Klemmhalterung ist gesichert. Zum Verschieben des Hautlappens wird die Vorrichtung am Griffrohr erfaßt und in die notwendige Verschieberichtung bewegt, dabei wird das entsprechende Klemmschenkelende entsprechend der auftretenden Zugspannung aus dem Griffrohr herausgezogen. Diese Längenänderungsanzeige korrespondiert

5

mit der Zugkraft bzw. mit der auftretenden Hautspannung.
Ein solches Gerät wird nur zur Messung der auftretenden
Hautspannung verwendet. Die eigentliche öperative Verschiebtechnik wird nach Feststellung der geeigneten Hautspannung
mit **einem** anderen chirurgischen Gerät ausgeführt.

Weitere Merkmale der Erfindung werden im folgenden anhand
der abgebildeten Ausführungsbeispiele erläutert. Es zeigen:

Fig. 1        die teilweise geschnittene Ansicht einer
              pinzettenartigen Vorrichtung und

Fig. 2        die teilweise geschnittene Ansicht einer
              zangenartigen Vorrichtung.

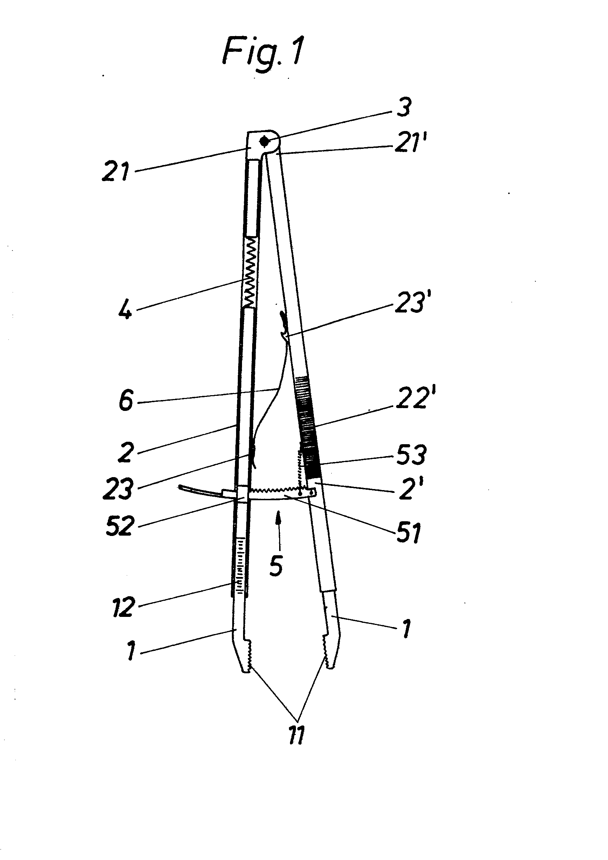

Zunächst wird auf Fig. 1 Bezug genommen. Das pinzettenartige Gerät besteht aus zwei an ihren abgeflachten Enden 21 bzw. 21' durch ein Gelenk 3 miteinander verbundenen
Rohren 2 bzw. 2'. In den freien Enden dieser Rohre sind
teleskopartig verschiebbar Klemmschenkel 1 geführt mit
verbreiterten Schenkelenden 11. Diese verbreiterten Enden
können an ihrer Innenseite rutschhemmende Elemente aufweisen, um die sichere Halterung der ergriffenen Hautlappen zu gewährleisten. Die Klemmschenkel 1 sind über

BAD ORIGINAL

6

zwischengeschaltete Zugfedern 4 kraftschlüssig mit den als Griffelementen ausgebildeten Rohren 2 bzw. 2' verbunden. Außen sind auf diesen Rohren zur Grifferleichterung beispielsweise Riffelungen 22' vorgesehen. Zumindest an einem Klemmschenkel 1 ist eine Längenänderungsanzeige 12 vorgesehen, die mit der Zugkraftsänderung korrespondiert. Je weiter der Klemmschenkel 1 aus dem Rohr 2 gegen den Zug der Feder 4 ausgezogen wird, um so größer ist die aufgebrachte Zugkraft und damit die Spannung an den ergriffenen und verschobenen Hautlappen.

Zur automatischen Spreizung der beiden Rohre 2 bzw. 2' dient die gewölbte Blattfeder 6, die an den Punkten 23 bzw. 23' festgesetzt ist.

Die insgesamt mit der Ziffer 5 bezeichnete Feststellvorrichtung besteht aus einer am Rohr 2' gegen eine Feder 53 pendelnd aufgehangenen Rastschiene 51 und einer am anderen Rohr 2 entsprechend vorgesehenen Rastführung 52. Im wesentlichen dient diese Vorrichtung 5 dazu, die beiden Rohre in der Klemmstellung festzusetzen, so daß nach dem Ergreifen des Hautlappens durch die Enden 11 die Klemmstellung gesichert und lediglich die Abzugskraft aufzubringen ist. Andere Feststellvorrichtungen sind möglich.

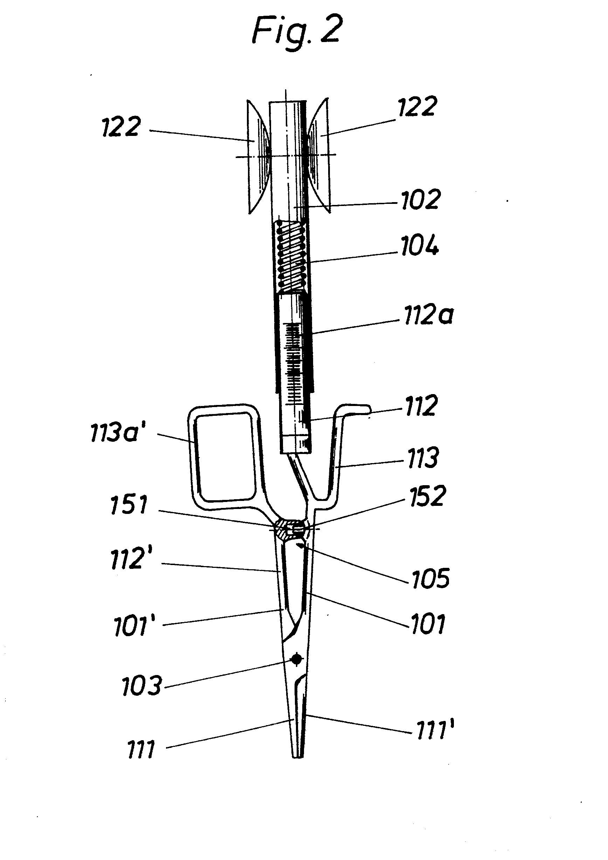

In Fig. 2 ist eine zangenartige Vorrichtung zum Prüfen der Zugspannung beim Verschieben von Hautlappen dargestellt. Die zangenartig verbundenen Klemmschenkel 101 bzw. 101' sind etwa mittig durch das Gelenk 103 verbunden. An den den verbreiterten Schenkelenden 111 bzw. 111' gegenüberliegenden Enden 112 bzw. 112' sind Griffelemente 113 und 113' angeordnet. An diesen Griffteilen werden die Klemmschenkel betätigt. In der Klemmstellung rastet ein am Schenkel 101 vorgesehener Rastzapfen 152 in die am anderen Schenkel 101' vorgesehene Rasthülse 151 einer insgesamt mit der Ziffer 105 bezeichneten Feststellvorrichtung 1 ein. Auch diese Feststellvorrichtung sichert den zwischen den Schenkelenden 111 und 111' eingeklemmten Hautlappen.

Zum Verschieben des Hautlappens wird die Vorrichtung ergriffen an den beiden Griffmulden 122, die an dem als Griffteil ausgebildeten Rohr 102 vorgesehen sind. In diesem Rohr 102 ist gegen den Zug einer Feder 104 das entsprechend ausgebildete freie Ende 112 des Klemmschenkels 101 wiederum in der Art einer Federwaage verschiebbar. An diesem zylinderförmigen Schenkelende 112 sind Längenmarkierungen vorgesehen, die die Zugkraftanzeige 112a bilden. Die aufgewandte Zugkraft korrespondiert mit der herausragenden Länge des Endes 112 aus dem Rohr 102.

BAD ORIGINAL

8

Statt der mechanischen mit Zugfedern arbeitenden Zugspannungsmessung können elektronisch messende und anzeigende Vorrichtungen eingesetzt werden. die beispielsweise auf dem Prinzip
der Dehnungsmessung mit Digitalanzeige arbeiten.

Zusammenstellung der Bezugszeichen

| | |
|---|---|
| 1 | Klemmschenkel |
| 11 | Schenkelende |
| 12 | Zugkraftanzeige |
| | |
| 2, 2' | Rohr |
| 21, 21' | abgeflachtes Rohrende |
| 22' | Riffelung |
| 23, 23' | Federbefestigung |
| | |
| 3 | Gelenk |
| | |
| 4 | Zugfeder |
| | |
| 5 | Feststellvorrichtung |
| 51 | Rastschiene |
| 52 | Rastführung |
| 53 | Zugfeder |
| | |
| 6 | Blattfeder |
| | |
| 101, 101' | Klemmschenkel |
| 111, 111' | Schenkelende |
| 112, 112' | Schenkelende |
| 112a | Zugkraftanzeige |
| 113, 113' | Griffteil |

0115624

– /11 –

| 102 | Rohr |
| 122 | Griffmulde |
| 103 | Gelenk |
| 104 | Zugfeder |
| 105 | Feststellvorrichtung |
| 151 | Rasthülse |
| 152 | Rastzapfen |

**Patentansprüche**  — / —

1. Vorrichtung zum Prüfen der Zugspannung beim Verschieben von Hautlappen, gekennzeichnet durch ein pinzetten- oder zangenartiges Gerät mit breitflächigen Enden (11, 111, 111') an den beiden Klemmschenkeln (1, 101, 101'), wobei die Klemmschenkel (1, 101) über Zugspannungsmesser(12, 112, 112a) einzeln oder gemeinsam kraftschlüssig mit einem Griffteil (2, 102) verbunden sind.

2. Vorrichtung nach Anspruch 1, gekennzeichnet durch zwei an einem Ende (21, 21') gelenkig miteinander verbundene Rohre (2, 2'), in denen jeweils teleskopartig ein Klemmschenkel (1) in Zugrichtung gegen eine im jeweiligen Rohr (2, 2') befestigte Feder (4) mit gleicher Federkennlinie verschiebbar ist, wobei eine mit der Zugkraft korrespondierende Längenänderungsanzeige (12) am Klemmschenkel (1) vorgesehen ist.

3. Vorrichtung nach Anspruch 2, gekennzeichnet durch eine Feststellvorrichtung (5) für die das Griffteil bildenden Rohre (2, 2') in der Klemmstellung.

4. Vorrichtung nach Anspruch 2, gekennzeichnet durch eine zwischen den Rohren (2, 2') angeordnete Spreizfeder (6).

BAD ORIGINAL

5. Vorrichtung nach Anspruch 1, gekennzeichnet durch zwei zangenartig gelenkig miteinander verbundene, in der Klemmstellung festsetzbare Klemmschenkel (101, 101'), von denen ein Schenkel (101) mit einem Ende (112) teleskopartig geführt in einem Griffrohr (102) in Zugrichtung gegen eine Feder (104) verschiebbar und eine mit der Zugkraft korrespondierende Längenänderungsanzeige (112 a) am Schenkel (102) vorgesehen ist.

6. Vorrichtung nach Anspruch 5, dadurch gekennzeichnet, daß an den Klemmschenkeln (101, 101') Griffelemente (113, 113') vorgesehen sind.

7. Vorrichtung nach Anspruch 5, daduruch gekennzeichnet, daß am Griffrohr (102) Griffmulden (122) oder dergleichen vorgesehen sind.

8. Vorrichtung nach Anspruch 5, dadurch gekennzeichnet, daß an einem Klemmschenkel (101) ein Rastzapfen (152) vorgesehen ist, der in eine Rasthülse (151) am anderen Klemmschenkel (101') eingreift und in einer oder mehreren Spreizstellungen der Schenkel (101, 101') zueinander einrastbar ist.

Fig.1

0115624

## Fig. 2